# EUROPÄISCHE PATENTANMELDUNG

(11) **EP 1 541 179 A1**
(43) Veröffentlichungstag der Anmeldung: **15.06.2005**
(21) Anmeldenummer: 03028382.4
(22) Anmeldetag: 10.12.2003
(51) Int. Cl.: A61L 9/20, F24F 3/16, F21V 7/00

(54) **Entkeimungsanlage**

(71) Anmelder: BÄ*RO GmbH & Co. KG, 42799 Leichlingen (DE)
(72) Erfinder: Kirsten, Martin Dr., 51399 Burscheid (DE)
(74) Vertreter: Paul, Dieter-Alfred, Dipl.-Ing.

(57) **Zusammenfassung**

Die Erfindung betrifft eine Entkeimungsanlage (2) für die Luftentkeimung von Klimaanlagen oder Umluftgeräten mit einem Strömungskanal (1) für die Durchführung der Luft und wenigstens einem UV-C-Strahler (4), um die den Strömungskanal (1) in einer Strömungsrichtung durchströmende Luft mit UV-C-Strahlung zu behandeln und so die darin enthaltenen Mikroorganismen abzutöten, welche dadurch gekennzeichnet ist, daß wenigstens ein UV-C-Strahler (4) außerhalb des Strömungskanals (1) angeordnet ist und in einen Strömungskanalabschnitt UV-C-Strahlung einstrahlt.

## Beschreibung

Die vorliegende Erfindung betrifft eine Entkeimungsanlage für die Luftentkeimung von Klimaanlagen oder Umluftgeräten mit einem Strömungskanal für die Durchführung der Luft und wenigstens einem UV-C-Strahler, um die den Strömungskanal in einer Strömungsrichtung durchströmende Luft mit UV-C-Strahlung zu behandeln und so die darin enthaltenen Mikroorganismen abzutöten.

Zur Entkeimung von Luft in gewerblich und industriell genutzten Räumen sowie in Krankenhäusern oder Flugzeugen werden UV-C-Strahler eingesetzt. Ihre keimtötende UV-C-Linienstrahlung mit einer Wellenlänge von λ = 253,7 nm wird verwendet, um Bakterien, Pilze, Viren oder andere Mikroorganismen abzutöten. Die UV-C-Strahler sind dabei in Luftreinigungsgeräten der eingangs genannten Art in einen Strömungskanal für die Durchführung der zu reinigenden Luft eingesetzt.

Nachteilig an den bekannten Luftreinigungsgeräten ist, daß die UV-C-Strahler dem zu entkeimenden Luftstrom einen hohen Luftwiderstand entgegensetzen. Des weiteren müssen die im Luftstrom befindlichen UV-C-Strahler vor eventueller Verschmutzung durch in der durchströmenden Luft befindliche Partikel geschützt werden, so daß eine Vorschaltung eines Filterelements unumgänglich ist. Hierdurch wird zum einen der Luftwiderstand weiter erhöht, und zum anderen kann es zu ungünstigen Verwirbelungen der Luft im Luftkanal führen. Ein weiteres Problem besteht darin, daß bei den bekannten Luftreinigungsgeräten die von den UV-C-Strahlern produzierte Wärme direkt an den Luftstrom abgegeben wird, wodurch die Temperatur in dem Luftstrom steigt. Um dieser Temperaturerhöhung entgegenzutreten, muß die erwärmte Luft mittels der Klimaanlage bzw. des Umluftgerätes wieder abgekühlt werden. Im Ergebnis haben die bekannten Luftreinigungsgeräte einen erheblichen Energieverbrauch.

Schließlich ist bei den herkömmlichen Entkeimungssystemen problematisch, daß beispielsweise bei Wartungsarbeiten, Reinigungsarbeiten oder einem Strahleraustausch bei Ausfall eines UV-C-Strahlers das komplette System der Klimaanlage oder des Umluftgerätes außer Betrieb gesetzt werden muß. Wird dieses Entkeimungssystem z.B. in der fleischverarbeitenden Industrie in Reiferäumen eingesetzt, führt ein zeitweiliger Betriebsausfall ggf. zu einer Unterbrechung der Kühlkette bzw. der zur Reifung notwendigen Umgebungsbedingungen und somit zu einem erhöhten Risiko für die Produkte oder zu einer notwendigen Prozeßunterbrechung des Reifeprozesses.

Werden elektronisch betriebene UV-C-Kontaktniederdruckstrahler eingesetzt, darf die maximale Leitungslänge zur Stromversorgung nur 2 m betragen. Die Montage des dafür notwendigen elektronischen Vorschaltgerätes außerhalb des Strömungskanals ist daher sehr schwierig, die Möglichkeiten zur Montage der gesamten Anlage dadurch sehr eingeschränkt. Werden die Vorschaltgeräte aufgrund der beschränkten Montagemöglichkeiten zusätzlich zu den UV-C-Strahlern direkt in den Strömungskanal eingesetzt, wirken diese als Strömungswiderstand und als Wärmeproduzent, so daß der Wirkungsgrad des Gesamtsystems verringert wird.

Im übrigen wird als nachteilig empfunden, daß sich bestehende Klimaanlagen nur mit erheblichem Aufwand mit den bekannten Entkeimungsanlagen nachrüsten lassen. Wenn eine solche Entkeimungsanlage in bereits bestehende Klimaanlagen eingebaut werden soll, muß nämlich ein komplettes Strömungskanalstück durch das UV-C-Bestrahlungsmodul ersetzt werden. Dies ist mit einem sehr großen Montageaufwand, mit einer Unterbrechung des Betriebes der Klimaanlage sowie mit erhöhten Materialkosten verbunden.

Es ist daher Aufgabe der vorliegenden Erfindung, ein Entkeimungsanlage bereitzustellen, welche die Luft von Klimaanlagen und/oder Umluftgeräten wirkungsvoll entkeimt, dabei den Wirkungsgrad der Klimaanlagen und/oder der Umluftgeräte möglichst wenig beeinflußt und einfach in bereits bestehende Systeme einbaubar ist.

Diese Aufgabe ist erfindungsgemäß dadurch gelöst, daß wenigstens ein UV-C-Strahler außerhalb des Strömungskanals angeordnet ist und in einen Strömungskanalabschnitt UV-C-Strahlung einstrahlt. Der Erfindung liegt damit die Überlegung zugrunde, die zur Bestrahlung der Luft in einer Klimaanlage oder in einem Umluftgerät notwendigen UV-C-Strahler außerhalb des Strömungskanals vorzusehen, um so den Strömungswiderstand im Strömungskanal und die auftretenden Luftverwirbelungen an den UV-C-Reflektoren zu reduzieren und die durch die UV-C-Strahler erzeugte Wärme nicht in den Luftstrom abzugeben, sondern an die Umgebung außerhalb des Strömungskanals abzuführen. Weiterhin wird die Gefahr von Verschmutzungen der Reflektoren durch eventuell in dem Luftstrom befindliche Partikel vermindert, so daß der Einbau eines vorgeschalteten Filters unterbleiben kann, was gleichzeitig zu einer Materialkostenreduktion und zu einem verringerten Energieaufwand aufgrund des geringeren Luftwiderstands im Klimasystem führt.

Bei einer Ausführungsform der Erfindung ist der UV-C-Strahler länglich, d. h. in Röhrenform, ausgebildet und erstreckt sich in Strömungsrichtung entlang des Strömungskanals. Auf diese Weise wird die Bestrahlungsstrecke entsprechend der Ausdehnung der UV-C-Strahler vergleichsweise lang, wodurch die Bestrahlungszeit der zu entkeimenden Luft sowie die Bestrahlung, welche sich aus dem Produkt aus Bestrahlungsstärke und Bestrahlungszeit ergibt, und damit die Entkeimungseffektivität hoch sind. Um den Strömungskanal optimal ausleuchten zu können, ist es möglich, mehrere UV-C-Strahler nebeneinander in Strömungsrichtung der Luft anzuordnen. Ebenso ist es möglich, die Bestrahlungsstrecke zu verlängern, indem mehrere Strahler in Strömungsrichtung hintereinander angeordnet werden.

Eine alternative oder zusätzliche Ausführungsform sieht zur weiteren Erhöhung der Bestrahlungsintensität sowie einer variablen Gestaltung und der Bestrahlungsstrecke entlang des Strömungskanals vor, einen oder mehrere länglich ausgebildete UV-C-Strahler quer zur Strömungsrichtung nebeneinander am Strömungskanal anzuordnen.

Zur Steigerung der Effektivität der Entkeimung durch die UV-C-Strahler und zur Erhöhung der Bestrahlungsintensität kann der UV-C-Strahler einen Teil der UV-C-Strahlung als Direktanteil und einen anderen Teil als einen durch eine den UV-C-Strahler umgebende Reflektoreinheit reflektierten Teil in den Strömungskanal einstrahlen.

Dabei kann diese Ausführungsform der Erfindung vorsehen, den Strömungskanal mit einem rechteckigen oder quadratischen Querschnitt auszubilden und zur Erzielung einer hohen UV-C-Bestrahlungsstärke an benachbarten Strömungskanalwänden des Strömungskanals UV-C-Strahler vorzusehen, die jeweils von einer Reflektoreinheit umgeben sind, wodurch ein UV-C-Strahlungsgitter durch die von den UV-C-Strahlern emittierte UV-C-Strahlung entsteht.

Die Reflektoreinheit kann dabei durch einen Parabolreflektor gebildet sein, der derart angeordnet ist, daß sich sein Brennpunkt in einem Entladungsrohr des UV-C-Strahlers befindet, wodurch sich eine parallele Ausbreitung der von dem UV-C-Strahler emittierten UV-C-Strahlung ergibt.

Werden die länglichen UV-C-Strahler quer zur Strömungsrichtung am Strömungskanal angeordnet, kann die Reflektoreinheit auch derart ausgebildet sein, daß sie eine Reflexion in Form einer Batwing-Verteilung erzeugt, und somit der reflektierte Teil der UV-C-Strahlung auch in benachbarte Strömungskanalabschnitte ausstrahlt. Dies führt vorteilhaft dazu, daß die effektive Bestrahlungsstrecke größer ist als der Bereich, in dem die UV-C-Strahler angeordnet sind und sich damit auch die Bestrahlungszeit bzw. die Bestrahlungsdosis der in dem Strömungskanal geführten Luft noch weiter vergrößert.

Eine bevorzugte Ausführungsform sieht vor, den UV-C-Strahler von einem Strahlergehäuse zu umgeben, in welches die Reflektoreinheit eingesetzt ist, wobei das Strahlergehäuse, die Reflektoreinheit und der UV-C-Strahler zusammen eine modulartige UV-C-Bestrahlungseinheit bilden, die an dem Strömungskanal anbringbar ist. Durch diesen modulartigen Aufbau der UV-C-Bestrahlungseinheiten ist eine Montage der UV-C-Strahler sowie eine Nachrüstung eines bereits bestehenden Klimasystems besonders einfach.

In bevorzugter Weise kann eine UV-C-transparente Quarzglasscheibe oder eine transparente Teflonfolie die UV-C-Strahler räumlich von dem Strömungskanal trennen. Dies hat zur Folge, daß keine zusätzlichen Verwirbelungen der den Strömungskanal durchströmenden Luft im Bereich der eingesetzten UV-C-Strahler auftreten und die UV-C-Strahler vor einer Verschmutzung oder einer Beschädigung durch eventuell im Luftstrom befindliche Partikel geschützt werden.

Um zu verhindern, daß ein in dem Klimasystem eingesetztes, gegen UV-C-Strahlung empfindliches Material durch die Direktstrahlung der UV-C-Strahlung beschädigt wird, können die sich an die zu bestrahlenden Strömungskanalabschnitte anschließenden Strömungskanalabschnitte eine UV-C-Strahlung absorbierende Oberfläche, eine sogenannte UV-C-Strahlungsfalle, aufweisen.

Als UV-C-Strahler können beispielsweise High-Output-UV-C-Kompaktniederdruckstrahler mit zwei Entladungsrohren vorgesehen sein. Bei Einsatz solcher UV-C-Strahler ist es vorteilhaft, die Reflektoreinheiten jeweils durch zwei Parabolreflektorflächen auszubilden, die derart angeordnet sind, daß sich ihre Brennpunkte jeweils in einem der Entladungsrohre des UV-C-Strahlers befinden, wodurch sich auch hier eine parallele Ausbreitung der von den Entladungsrohren des UV-C-Strahlers emittierten UV-C-Strahlung ergibt.

Eine weitere Möglichkeit der Erhöhung der Strahlungsintensität besteht darin, wenigstens eine Innenwand des Strömungskanals, insbesondere die den UV-C-Strahlern jeweils gegenüberliegende Wand, mit einer UV-C-Strahlung reflektierenden Oberfläche auszubilden. So kann die UV-C-Strahlung, die den Strömungskanal durchlaufen hat, ohne von einem Mikroorganismus absorbiert worden zu sein, durch Reflexion der Strahlung der an der dem emittierenden UV-C-Strahler gegenüberliegenden Wand den Strömungskanal ein weiteres Mal in entgegengesetzter Richtung durchlaufen.

Hinsichtlich weiterer vorteilhafter Ausgestaltungen und Weiterbildungen der Erfindung wird auf die Unteransprüche sowie die nachfolgende Beschreibung von Ausführungsbeispielen anhand der beiliegenden Zeichnung verwiesen. In der Zeichnung zeigt:
- Figur 1: eine perspektivische Darstellung einer ersten Ausführungsform einer erfindungsgemäßen Entkeimungsanlage, bei der modulartige UV-C-Bestrahlungseinheiten entlang eines UV-C-Strömungskanals angeordnet sind;
- Figur 2: einen Längsschnitt durch eine zweite Ausführungsform einer erfindungsgemäßen Entkeimungsanlage mit mehreren quer zur Strömungsrichtung außerhalb des Strömungskanals angeordneten, modulartigen UV-C-Bestrahlungseinheiten angebracht ist;
- Figur 3: eine modulartige UV-C-Bestrahlungseinheit mit einem High-Output-UV-C-Kompaktniederdruckstrahler, bei der eine Reflektoreinheit durch zwei Parabolreflektoren gebildet ist, im Querschnitt;
- Figur 4: im Querschnitt eine dritte Ausführunsform einer erfindungsgemäßen Entkeimungsanlage, bei welcher die UV-C-Bestrahlungseinheiten gemäß Figur 3 derart angeordnet sind, daß sich ein Strahlungsgitter ergibt;
- Figur 5: in schematischer Darstellung eine UV-C-Bestrahlungseinheit, die eine Reflektoreinheit aufweist, welche eine Batwing-Verteilung erzeugt; und
- Figur 6: einen Längsschnitt durch eine vierte Ausführunsform einer erfindungsgemäßen Entkeimungsanlage, bei welcher Reflektoren gemäß Figur 5 quer zur Strömungsrichtung an ein Strömungskanal angeordnet sind.

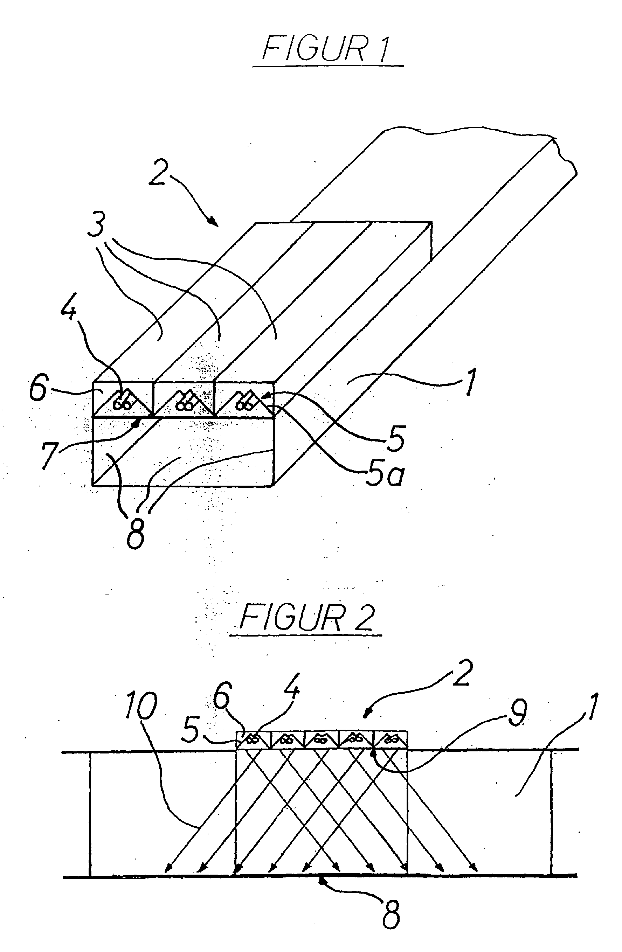

Die Figur 1 zeigt eine perspektivische Darstellung einer ersten Ausführungsform einer erfindungsgemäßen Luftentkeimungsanlage 2. Die Entkeimungsanlage 2 besitzt einen von Luft durchströmten Strömungskanal 1 mit einem rechteckigen Querschnitt, an dessen Oberseite drei modulartige UV-C-Bestrahlungseinheiten 3 vorgesehen sind. Diese Bestrahlungseinheiten 3 sind außerhalb des Strömungskanals 1 angeordnet, so daß sie dem Luftstrom darin keinen widerstand entgegensezten, und umfassen jeweils einen länglich ausgebildeten High-Output-UV-C-Kompaktniederdruckstrahler 4, einen den Strahler 4 umgebenden UV-C-Reflektor 5 mit unter 45° zur Mittellinie geknickten Reflektorflächen 5a und ein den UV-C-Strahler 4 und den Reflektor 5 einschließendes Strahlergehäuse 6, das zum Strömungskanal 1 hin offen ist, so daß die Strahler 4 UV-C-Strahlung in den Kanal 1 einstrahlen. Dabei wird ein Teil der Strahlung als Direktanteil, und der Rest als ein von den Reflektorflächen 5a der Reflektoreinheit 5 reflektierter Teil eingestrahlt. Die UV-C-Bestrahlungseinheiten 3 sind durch eine UV-C-transparente Quarzglasscheibe 7 räumlich von dem Strömungskanal 1 getrennt, so daß eine eventuelle ungünstige Verwirbelung der den Strömungskanal 1 durchströmenden Luft im Bereich der UV-C-Bestrahlungseinheiten 3 vermieden wird. Die nicht mit UV-C-Bestrahlungseinheiten 3 besetzten Innenflächen des Strömungskanals 1 weisen eine UV-C-Strahlung reflektierende Oberfläche 8 auf.

In der Figur 2 ist eine zweite Ausführungsform einer erfindungsgemäßen Entkeimungsanlage 2 dargestellt. In dieser Ausführungsform sind UV-C-Bestrahlungseinheiten 3 quer zur Strömungsrichtung außerhalb und an der Oberseite eines ebenfalls rechteckig ausgebildeten Strömungskanals 1 angeordnet. Auch hier bestehen die UV-C-Bestrahlungseinheiten 3 - wie schon in Figur 1 - jeweils aus einem länglich ausgebildeten High-Output-UV-C-Kompaktniederdruckstrahler 4, einem UV-C-Reflektor 5 mit unter 45° zur Mittellinie geknickten Reflektorflächen 5a sowie einem Strahlergehäuse 6. Durch in Strömungsrichtung hintereinander liegende Anordnung der UV-C-Bestrahlungs-einheiten 3 wird die Bestrahlungsstrecke und damit auch die Bestrahlungszeit durch die Anzahl der UV-C-Bestrahlungseinheiten 3 festgelegt und kann durch Variation der Anzahl der UV-C-Bestrahlungseinheiten 3 verlängert oder verkürzt werden. In dem dargestellten Ausführungsbeispiel sind die UV-C-Bestrahlungs-einheiten 3 zur Verringerung von Luftverwirbelungen durch eine für UV-C-Strahlung transparente Teflonfolie 9 räumlich von dem Strömungskanal 1 getrennt. Die Richtungspfeile 10 zeigen die durch die Reflektoreinheit 5 hervorgerufene Ausbreitung der direkten sowie der reflektierten, indirekten UV-C-Strahlung an.

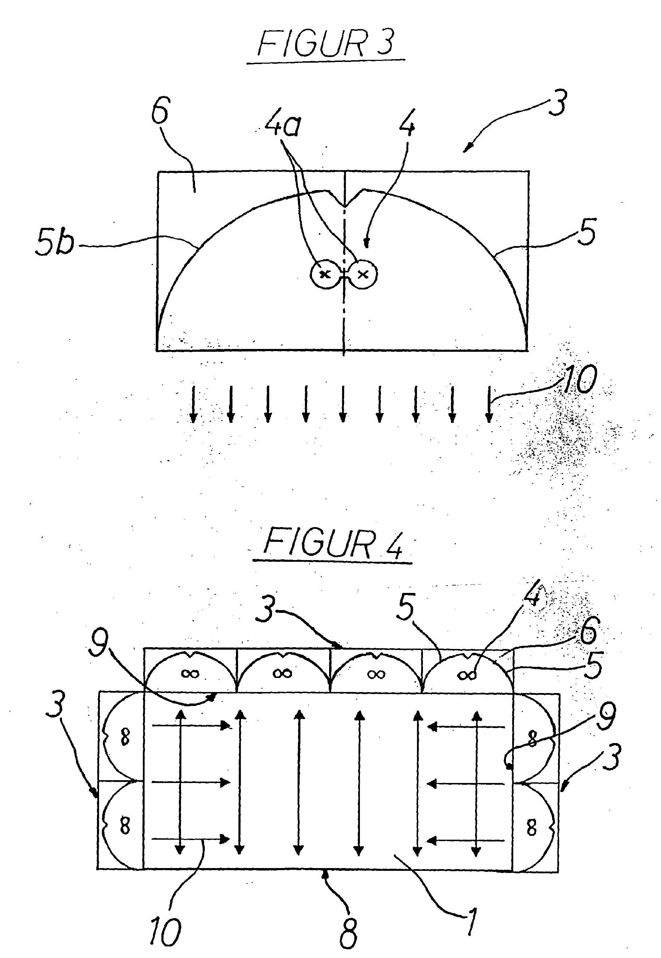

Die Figur 3 zeigt einen Querschnitt durch eine alternative Ausführungsform einer UV-C-Bestrahlungseinheit 3, bei der eine Reflektoreinheit 5 durch zwei Parabolreflektorflächen 5b ausgebildet ist, die einen High-Output-UV-C-Kompaktniederdruckstrahler 4 umgeben. Der High-Öutput-UV-C-Kompaktniederdruckstrahler 4 weist zwei Entladungsrohre 4a auf, die derart waagerecht in der UV-C-Bestrahlungseinheit 3 ausgerichtet sind, daß sie sich jeweils über ihre gesamte Ausdehnung in den beiden Brennpunkten der Parabolreflektorflächen 5b befinden, wodurch sich die durch die Richtungspfeile 10 angezeigte, parallele Ausbreitung der von dem UV-C-Strahler emittierten, direkten sowie reflektierten, indirekten UV-C-Strahlung ergibt.

Eine weitere Ausführungsform einer Entkeimungsanlage 2 ist in Figur 4 gezeigt. Die Entkeimungsanlage 2 umfaßt mehrere länglich ausgebildete und entlang eines Strömungskanals 1 angeordnete UV-C-Bestrahlungseinheiten 3, die gemäß Figur 3 ausgebildet sind, wobei jeweils zwei UV-C-Bestrahlungsein-heiten 3 an den beiden Seiten und vier UV-C-Bestrahlungseinheiten 3 an der Oberseite und außerhalb des Strömungskanals 1 angeordnet sind. Die UV-C-Bestrahlungseinheiten 3 sind durch für UV-C-Strahlung transparente Teflonfolien 9 zur Reduktion von Luftverwirbelungen räumlich von dem Strömungskanal 1 getrennt. Eine nicht mit UV-C-Bestrah-lungseinheiten 3 bzw. mit einer Teflonfolie 9 versehene Wand weist innenseitig eine UV-C-Strahlung reflektierende Oberfläche 8 auf. Wie durch die Richtungspfeile 10 angezeigt, ergibt sich bei dieser Anordnung durch die von den UV-C-Bestrahlungseinheiten 3 jeweils emittierte, parallele UV-C-Strahlung sowie durch die von der Oberfläche 8 reflektierte UV-C-Strahlung ein UV-C-Strahlungsgitter. Die Ausbreitungsrichtung der seitlich angeordneten UV-C-Bestrahlungseinheiten 3 steht dabei nahezu senkrecht auf der Ausbreitungsrichtung der UV-C-Strahlung, die von den im oberen Bereich des Strömungskanals 1 angeordneten Bestrahlungseinheiten 3 emittiert wird, wodurch sich eine besonders homogene und intensive Durchflutung des Strömungskanal mit UV-C-Strahlung ergibt.

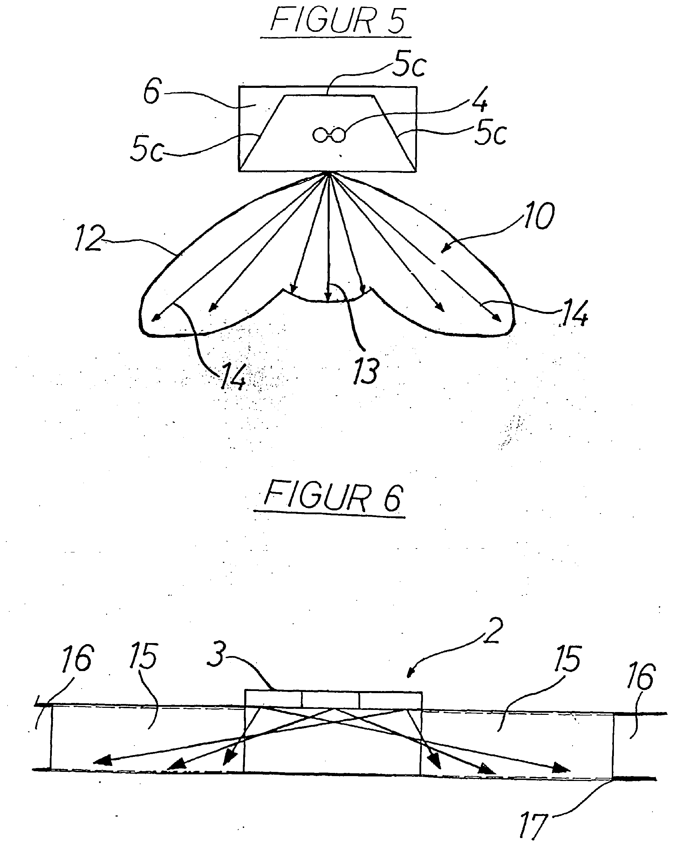

In der Figur 5 ist eine dritte Ausführungsform einer UV-C-Bestrahlungseinheit 3 gezeigt. Die UV-C-Bestrahlungseinheit 3 weist eine Reflektoreinheit 5 mit Reflektorflächen 5c auf, welche derart ausgebildet sind, daß sich eine UV-C-Strahlungsverteilungskurve 12 in Form einer Batwing-Verteilung ergibt. Diese UV-C-Strahlungsverteilungskurve 12 setzt sich aus einem Direktstrahlungsanteil 13 und einem reflektierten Strahlungsanteil 14 zusammen.

Durch diese Strahlungsverteilung 12 wird bei einer in Figur 6 gezeigten Anordnung von länglich ausgebildeten UV-C-Bestrahlungseinheiten 3 quer zur Strömungsrichtung erreicht, daß die von den UV-C-Bestrahlungseinheiten 3 gemäß Figur 5 emittierte UV-C-Strahlung auch in benachbarte Strömungskanalabschnitte 15 ausstrahlt, wodurch die Bestrahlungsstrecke und somit auch die Bestrahlungszeit zusätzlich vergrößert werden und über die durch die UV-C-Bestrahlungseinheiten 3 gebildete Strecke hinausreicht und weit in die nicht mit UV-C-Bestrahlungseinheiten 3 besetzten benachbarten Strömungskanalabschnitte 15 einstrahlt.

Um gegen UV-C-Strahlung empfindliches Material im Strömungskanal 1 vor einer Schädigung, zu schützen, ist es wie in Figur 6 gezeigt notwendig, die sich an die zu bestrahlenden Strömungskanalabschnitte 15 anschließenden Strömungskanalabschnitte 16 mit einer sogenannten Strahlungsfalle auszubilden. Die Strömungskanalasbschnitte 16 weisen dazu an ihren Innenseiten eine UV-C-Strahlung absorbierenden Oberfläche 17 auf.

## Patentansprüche

1. Entkeimungsanlage (2) für die Luftentkeimung von Klimaanlagen oder Umluftgeräten mit einem Strömungskanal (1) für die Durchführung der Luft und wenigstens einem UV-C-Strahler (4), um die den Strömungskanal (1) in einer Strömungsrichtung durchströmende Luft mit UV-C-Strahlung zu behandeln und so die darin enthaltenen Mikroorganismen abzutöten, **dadurch gekennzeichnet, daß** wenigstens ein UV-C-Strahler (4) außerhalb des Strömungskanals (1) angeordnet ist und in einen Strömungskanalabschnitt UV-C-Strahlung einstrahlt.

2. Entkeimungsanlage (2) nach Anspruch 1, **dadurch gekennzeichnet, daß** der UV-C-Strahler (4) länglich ausgebildet ist und sich in Strömungsrichtung entlang des Strömungskanals (1) erstreckt.

3. Entkeimungsanlage (2) nach Anspruch 1, **dadurch gekennzeichnet, daß** der UV-C-Strahler (4) länglich ausgebildet und quer zur Strömungsrichtung am Strömungskanal (1) angeordnet ist.

4. Entkeimungsanlage (2) nach einem der Ansprüche 1 bis 3, **dadurch gekennzeichnet, daß** der UV-C-Strahler (4) einen Teil der UV-C-Strahlung als Direktanteil und einen anderen Teil durch eine den UV-C-Strahler (4) umgebende Reflektoreinheit (5) als reflektierten Teil in den Strömungskanal (1) einstrahlt.

5. Entkeimungsanlage (2) nach Anspruch 4, **dadurch gekennzeichnet, daß** der Strömungskanal (1) einen rechteckigen oder quadratischen Querschnitt aufweist, und zur Erzielung einer hohen UV-C-Bestrahlungsstärke an benachbarten Strömungskanalwänden des Strömungskanals (1) UV-C-Strahler (4) vorgesehen sind, die jeweils von einer Reflektoreinheit (5) umgeben sind, wodurch ein UV-C-Strahlungsgitter durch die von den UV-C-Strahlern emittierte UV-C-Strahlung entsteht.

6. Entkeimungsanlage (2) nach Anspruch 4 oder 5, **dadurch gekennzeichnet, daß** die Reflektoreinheit (5) durch wenigstens eine Parabolreflektorfläche (5b) gebildet ist, die derart angeordnet ist, daß sich ihr Brennpunkt jeweils in einem Entladungsrohr (4a) des UV-C-Strahlers (4) befindet, wodurch sich eine parallele Ausbreitung der von dem UV-C-Strahler (4) emittierenden UV-C-Strahlung ergibt.

7. Entkeimungsanlage (2) nach Anspruch 3 und 4 oder 5, **dadurch gekennzeichnet, daß** die Reflektoreinheit (5) derart ausgebildet ist, daß sie eine Reflexion in Form einer Batwing-Verteilung erzeugt, so daß der reflektierte Teil (14) der UV-C-Strahlung auch in benachbarte Strömungskanalabschnitte (15) ausstrahlt.

8. Entkeimungsanlage (2) nach einem der Ansprüche 4 bis 7, **dadurch gekennzeichnet, daß** der UV-C-Strahler (4) von einem Strahlergehäuse (6) umgeben ist, in welches die Reflektoreinheit (5) eingesetzt ist, wobei das Strahlergehäuse (6), die Reflektoreinheit (5) und der UV-C-Strahler (4) eine modulartige Bestrahlungseinheit (3) bilden, die an dem Strömungskanal (1) anbringbar ist.

9. Entkeimungsanlage (2) nach Anspruch 8, **dadurch gekennzeichnet, daß** mehrere Bestrahlungseinheiten (3) nebeneinander entlang und/oder quer zur Strömungsrichtung der Luft angeordnet sind.

10. Entkeimungsanlage (2) nach einem der vorhergehenden Ansprüche, **dadurch gekennzeichnet, daß** eine UV-C-transparente Quarzglasscheibe (7) oder eine transparente Teflonfolie (9) die UV-C-Strahler (4) räumlich von dem Strömungskanal (1) trennt.

11. Entkeimungsanlage (2) nach einem der vorhergehenden Ansprüche, **dadurch gekennzeichnet, daß** die sich an die zu bestrahlenden Strömungskanalabschnitte (15) anschließenden Strömungskanalabschnitte (16) eine UV-C-Strahlung absorbierende Oberfläche (17) aufweisen.

12. Entkeimungsanlage (2) nach einem der vorhergehenden Ansprüche, **dadurch gekennzeichnet, daß** der UV-C-Strahler (4) ein High-Output-UV-C-Kompaktniederdruckstrahler mit zwei Entladungsrohren (4a) ist.

13. Entkeimungsanlage (2) nach Anspruch 12, **dadurch gekennzeichnet, daß** die Reflektoreinheit (5) durch zwei Parabolreflektorflächen (5b) gebildet ist, die derart angeordnet sind, daß sich ihre Brennpunkte jeweils in einem der Entladungsrohre (4a) des UV-C-Strahlers (4) befinden, wodurch sich eine parallele Ausbreitung der von dem UV-C-Strahler (4) emittierten UV-C-Strahlung ergibt.

14. Entkeimungsanlage (2) nach einem der vorhergehenden Ansprüche , **dadurch gekennzeichnet, daß** wenigstens eine Innenwand des Strömungskanals (1) insbesondere die den UV-C-Strahlern (4) jeweils gegenüberliegende Innenwand, eine UV-C-Strahlung reflektierende Oberfläche (8) aufweist.
